# EUROPÄISCHE PATENTSCHRIFT

(11) **EP 3 073 968 B1**
(45) Veröffentlichungstag und Bekanntmachung des Hinweises auf die Patenterteilung: **08.11.2017**
(21) Anmeldenummer: 14796498.5
(22) Anmeldetag: 11.11.2014
(51) Int. Cl.: A61F 2/46, A61B 17/17, A61F 2/30, A61B 17/56, A61B 90/00

(54) **MEDIZINISCHES INSTRUMENTARIUM**
MEDICAL INSTRUMENT SET
INSTRUMENT MÉDICAL

(30) Priorität: 13.11.2013 DE 102013112497
(43) Veröffentlichungstag der Anmeldung: 05.10.2016
(73) Patentinhaber: Aesculap AG, 78532 Tuttlingen (DE)
(72) Erfinder: RICHTER, Berna, 78570 Mühlheim (DE); WINDHAGEN, Henning, 30559 Hannover (DE)
(74) Vertreter: Hoeger, Stellrecht & Partner Patentanwälte mbB
(86) Internationale Anmeldenummer: PCT/EP2014/074253
(87) Internationale Veröffentlichungsnummer: WO 2015/071252

(56) Entgegenhaltungen:
- WO-A1-2012/010366
- WO-A1-2012/021264
- US-A1- 2011 224 674

## Beschreibung

Die vorliegende Erfindung betrifft ein medizinisches Instrumentarium zum Implantieren einer Hüftgelenkpfanne umfassend mindestens ein medizinisches Führungsinstrument für ein erstes Fräswerkzeug, wobei das Führungsinstrument einen Formkörper umfasst, welcher Formkörper eine patientenindividuelle, in distaler Richtung weisende und von einem Kugeloberflächenausschnitt abweichende Knochenanlagefläche aufweist, welche Knochenanlagefläche korrespondierend oder im Wesentlichen korrespondierend zu einer Knochenoberfläche des Patienten geformt ist, wobei vom Formkörper in proximaler Richtung weisend ein eine Längsachse definierender Führungsschaft zum Führen des ersten Fräswerkzeugs absteht und wobei das medizinische Instrumentarium ein erstes Fräswerkzeug umfasst.

Insbesondere bei Patienten mit Hüftdysplasien erschweren die dabei vorliegenden Deformitäten des Beckenknochens eine ideale Positionierung einer Hüftgelenkendoprothese. Da es nicht möglich ist, das vorhandene pathologische Acetabulum als Pfannenlager aufzufräsen, muss ein annähernd anatomisches Pfannenlager rekonstruiert werden. Dieses kann jedoch von der vorliegenden pathologischen Situation deutlich abweichen. Zwar kann präoperativ eine optimale Position geplant werden, intraoperativ hat der Arzt jedoch keine Möglichkeit, das visuell Geplante definiert zu übertragen. Hier ist er im Wesentlichen auf seine Erfahrung angewiesen.

Die US 2011/0224674 A1 offenbart patientenspezifische Ausrichtinstrumente für das Acetabulum. In der WO 2012/010366 A1 und der WO 2012/021264 A1 sind Führungsinstrumente für Acetabulumfräser beschrieben.

Es ist daher eine Aufgabe der vorliegenden Erfindung, ein medizinisches Instrumentarium der eingangs beschriebenen Art so zu verbessern, dass auch bei Patienten mit Hüftdysplasien Hüftgelenkendoprothesen möglichst ideal positioniert werden können.

Diese Aufgabe wird bei einem medizinischen Instrumentarium der eingangs beschriebenen Art erfindungsgemäß dadurch gelöst, dass das erste Fräswerkzeug in Form eines Eingangsfräsers ausgebildet ist mit einem kanulierten, eine Fräserschaftlängsachse definierenden Fräserschaft, welcher ausgebildet ist zur Aufnahme des Führungsschafts des medizinischen Führungsinstruments.

Ein medizinisches Instrumentarium mit einem derart ausgebildeten Führungsinstrument ermöglicht es insbesondere, ein erstes Fräswerkzeug, beispielsweise einen Eingangs- oder Eröffnungsfräser, an der richtigen Stelle und mit der gewünschten Orientierung anzusetzen, um das zu rekonstruierende Pfannenlager auszubilden. Da der Arzt intraoperativ häufig eine schlechte Sicht auf den Operationssitus hat, bietet ihm das Führungsinstrument eine gute Unterstützung, um die geplante und gewünschte Position des Pfannenlagers in situ zu finden und diese während des Fräsvorgangs auch beizubehalten. So kann vermieden werden, dass vorhandene Deformitäten dazu führen, dass der Fräser abgleitet oder durch knöcherne Strukturen in unerwünschte Positionen gedrängt wird, ohne dass der Arzt dies merkt beziehungsweise ohne er es schafft, dagegen zu halten. Die patientenindividuelle Knochenanlagefläche ermöglicht es insbesondere, den Formkörper eindeutig am Beckenknochen des Patienten anzusetzen und dann das erste Fräswerkzeug mit dem Führungsschaft in die gewünschte Position zu führen, um das geplante Pfannenlager auszubilden. Das optimale Pfannenlager kann beispielsweise präoperativ auf Basis eines oder mehrerer Röntgenbilder geplant werden. Ausgehend hiervon kann beispielsweise die patientenindividuelle Knochenanlagefläche ausgebildet werden. Das vorgeschlagene Führungsinstrument ist insbesondere zum Einsatz bei adipösen Patienten gut geeignet, da bei diesen das Ertasten von Landmarken schwierig und damit fehleranfällig ist. Das Führungsinstrument ermöglicht es ferner, dass erste Fräswerkzeug mechanisch zu führen. Bekannte Systeme, die dem Operateur lediglich eine optische Führung auf einem Bildschirm bieten, insbesondere auch mit Unterstützung eines Navigationssystems, können jedoch nicht das Abrutschen des Fräswerkzeugs am Beckenknochen verhindern. Außerdem ermöglicht es das Führungsinstrument, insbesondere dann, wenn es einen durchbrechungsfreien Formkörper aufweist, eine zusätzliche Verletzung des Acetabulum-Bodens zu vermeiden. Wird beispielsweise der Formkörper mit einem Pin oder K-Draht fixiert, kann dieser beim Überfräsen durch überhöhte Reibung oder Verhaken tiefer ins Becken gebohrt werden und damit schwere Verletzungen verursachen. Dies wird mit dem vorgeschlagenen Führungsinstrument wirksam verhindert. Zudem kann der Führungsschaft so stabil ausgebildet werden, also ein hinreichend großen Durchmesser aufweisen, um eine hinreichend stabile mechanische Führung zu bilden, der also ein Abknicken oder Verbiegen beim Fräsen verhindern kann. Günstig ist es, dass das erste Fräswerkzeug in Form eines Eingangsfräsers ausgebildet ist mit einem kanulierten, eine Fräserschaftlängsachse definierenden Fräserschaft, welcher ausgebildet ist zur Aufnahme des Führungsschafts des medizinischen Führungsinstruments. Das derart ausgebildete erste Fräswerkzeug ermöglicht es, über den Führungsschaft geschoben zu werden und quasi eine erste Eingangsbohrung oder Eröffnungsbohrung am Acetabulum auszubilden. Eine Bewegung des ersten Fräswerkzeugs in distaler Richtung wird vorzugsweise durch den Formkörper des Führungsschafts begrenzt. Für eine optimale Führung entspricht ein Innendurchmesser einer Längsdurchbrechung des kanulierten Fräserschafts im Wesentlichen einem Außendurchmesser des Führungsschafts.

Günstig ist es, wenn die patientenindividuelle Knochenanlagefläche in distaler Richtung weisend angeordnet oder ausgebildet ist und wenn der Führungsschaft in proximaler Richtung weisend angeordnet oder ausgebildet ist. Das Führungsinstrument kann insgesamt einstückig ausgebildet sein. Denkbar ist auch eine mehrteilige Ausbildung. Beispielsweise wäre es denkbar, den Formkörper mit dem Schaft einteilig anzubieten und ein separates, auf den Formkörper aufsetzbares Element vorzusehen, welches die Knochenanlagefläche umfasst. Auf diese Weise kann die Herstellung des patientenindividuellen Führungsinstruments vereinfacht werden. Ein aus einem Stück geformtes Führungsinstrument ist besonders stabil.

Vorteilhaft ist es, wenn der Formkörper einen in distaler Richtung wirkenden Anschlag für ein Fräswerkzeug aufweist. Auf diese Weise kann genau vorgegeben werden, wie weit das Fräswerkzeug in Richtung auf das Acetabulum des Patienten hin bewegt werden kann. Damit kann auch die Tiefe des zu fräsenden Pfannenlagers vorgegeben werden.

Auf besonders einfache Weise lässt sich ein Anschlag ausbilden, wenn dieser eine in proximaler Richtung weisende Anschlagfläche aufweist. An dieser kann dann beispielsweise das erste Fräswerkzeug in Anlage kommen und ein weiteres Vordringen in das Becken verhindern.

Günstigerweise ist die Anschlagfläche eben oder im Wesentlichen eben. Diese ist besonders einfach herzustellen und hat zudem den Vorteil, dass eine Reibung zwischen der Anschlagfläche und dem Fräswerkzeug minimiert werden kann.

Um das Führen des Fräswerkzeugs weiter zu verbessern, ist es vorteilhaft, wenn die Anschlagfläche eine Ebene definiert, welche quer zur Längsachse verläuft. Insbesondere kann die Ebene senkrecht zur Längsachse verlaufen. Damit kann die gewünschte Orientierung für das auszubildende Pfannenlager genau vorgegeben werden. Insbesondere ist es günstig, wenn der Führungsschaft so lang ist, dass er insbesondere bei einem minimalinvasiven Zugang aus dem Körper des Patienten herausragt, wenn die patientenindividuelle Knochenanlagefläche am Acetabulum des Patienten anliegt.

Gemäß einer weiteren bevorzugten Ausführungsform der Erfindung kann vorgesehen werden sein, dass die Anschlagfläche an die patientenindividuelle Knochenanlagefläche angrenzt und dass ein Übergang zwischen der Anschlagfläche und der patientenindividuellen Knochenanlagefläche tangentenunstetig ist. Eine solche Ausgestaltung lässt sich besonders einfach herstellen. Beispielsweise kann so ein proximales Ende des Formkörpers abgefräst oder abgeschliffen werden zur Ausbildung der Anschlagfläche.

Vorteilhaft ist es, wenn eine Breite der Anschlagfläche in Abhängigkeit eines Umfangswinkels variiert. Dies gestattet ist einem Operateur, sofort zu erkennen, dass die Knochenanlagefläche eine eindeutige Form aufweist, so dass der Formkörper nur in einer ganz bestimmten Weise formschlüssig an das Acetabulum angelegt werden kann.

Um eine besonders gute Führung für das Fräswerkzeug zu erhalten, ist es vorteilhaft, wenn die Knochenanlagefläche mindestens teilweise an eine Kontur des Acetabulums des Patienten angepasst ist. Vorzugsweise ist sie vollständig an die Kontur des Acetabulums angepasst, und zwar in einem Flächenbereich den der Formkörper abdeckt.

Vorzugsweise weist die Knochenanlagefläche eine Kontur auf, welche mindestens einem Teil der Fossa und/oder einem Teil der Incisura Acetabuli entspricht. Beispielsweise kann so der Formkörper in Form eines negativen Abdrucks der Fossa und/oder eines Teils der Incisura Acetabuli ausgebildet werden.

Besonders einfach und kostengünstig herstellen lässt sich das Instrumentarium, wenn der Formkörper schalenförmig ausgebildet ist und eine konstante oder im Wesentlichen konstante Dicke aufweist.

Vorzugsweise ist die Knochenanlagefläche und/oder der Formkörper durch Gießen oder 3D-Drucken hergestellt ist. Zum Gießen des Formkörpers kann insbesondere eine auf Basis von patientenindividuellen Konturdaten des pathologischen Acetabulums Gießform hergestellt werden, beispielweise aus einem Metall oder einem Kunststoff. Beim 3D-Drucken der Knochenanlagefläche und/oder des Formkörpers können direkt patientenindividuelle, nichtinvasiv bestimmte Konturdaten zur Ausbildung der Knochenanlagenfläche und/oder des Formkörpers genutzt werden.

Gemäß einer weiteren bevorzugten Ausführungsform der Erfindung kann vorgesehen sein, dass die Knochenanlagefläche des Formkörpers Knochenanlageflächenkonturdaten definiert, welche nichtinvasiv bestimmten Knochenkonturdaten des Patienten entsprechen oder im Wesentlichen entsprechen. Insbesondere können die Knochenkonturdaten des Patienten aus Röntgen- oder Magnetresonanzaufnahmen des Patienten stammen. Mit anderen Worten wird die Knochenanlagefläche des Formkörpers insbesondere genau so ausgebildet, dass sie eine negative Form des pathologischen Acetabulums in dem Bereich bildet, wo das geplante Pfannenlager für die Hüftgelenkendoprothese ausgebildet werden soll.

Günstig ist es, wenn der Führungsschaft unlösbar oder lösbar verbindbar mit dem Formkörper verbunden ist. Die unlösbare Variante hat den Vorteil, dass die Ausrichtung des Führungsschafts relativ zur Knochenanlagefläche nicht versehentlich verändert werden kann. Eine lösbare Verbindbarkeit ermöglicht es jedoch, den Führungsschaft abhängig vom Patienten kürzer oder länger zu wählen.

Vorteilhaft ist es, wenn das Instrumentarium eine Kopplungseinrichtung zum temporären Koppeln des Formkörpers mit dem Führungsschaft aufweist. Mit der Kopplungseinrichtung können der Führungsschaft und der Formkörper auf einfache Weise verbunden oder voneinander gelöst werden.

Die Herstellung des Instrumentariums vereinfacht sich insbesondere dadurch, dass die die Kopplungseinrichtung mindestens ein erstes Kopplungselement und mindestens ein zweites Kopplungselement umfasst, dass die mindestens einen ersten und zweiten Kopplungselemente korrespondierend zueinander ausgebildet sind und in einer Kopplungsstellung in Eingriff stehen und in einer Lösestellung außer Eingriff stehen. Die ersten und zweiten Kopplungselemente können so einfach und sicher in definierter Weise miteinander verbunden werden.

Einfach und sicher lassen sich die ersten und zweiten Kopplungselemente koppeln, wenn das mindestens eine erste Kopplungselement in Form einer Kopplungsaufnahme und das mindestens eine zweite Kopplungselement in Form eines Kopplungsvorsprungs ausgebildet sind.

Ferner kann es günstig sein, wenn das mindestens eine erste Kopplungselement einen Innengewindeabschnitt und das mindestens eine zweite Kopplungselement einen korrespondierenden Außengewindeabschnitt umfasst. So können die beiden Kopplungselemente auf einfache Weise miteinander gekoppelt werden durch Verschrauben. Mit anderen Worten kann so beispielsweise der Führungsschaft an den Formkörper angeschraubt werden.

Vorteilhaft ist es, wenn das mindestens eine erste Kopplungselement am Formkörper angeordnet oder ausgebildet ist und wenn das mindestens eine zweite Kopplungselement am Führungsschaft angeordnet oder ausgebildet ist. Dies gestattet es, den Formkörper und den Führungsschaft bei Bedarf miteinander zu verbinden und auch wieder voneinander zu lösen.

Günstigerweise ist der Führungsschaft in Form eines langgestreckten zylindrischen Stabes ausgebildet. Ein solcher Führungsschaft ermöglicht eine einfache und sichere Führung eines insbesondere rotierenden Fräswerkzeugs.

Vorzugsweise umfasst das Instrumentarium ein erstes Fräswerkzeug und/oder ein zweites Fräswerkzeug. Die beiden Fräswerkzeuge können insbesondere unterschiedlich ausgebildet sein, beispielsweise zur Vorbereitung des Acetabulums zum Einsetzen unterschiedlicher Pfannen der Hüftgelenkendoprothesen.

Vorteilhaft ist es, wenn der Eingangsfräser einen Fräskopf aufweist, wenn der Fräskopf eine in sich geschlossene Bearbeitungsfläche aufweist und wenn die Bearbeitungsfläche einen Ausschnitt einer Kugeloberfläche bildet. Mit einem solchen Fräskopf lässt sich eine Eingangsbohrung mit einer Kontur am pathologischen Acetabulum ausbilden, welche einer Ringfläche einer Kugeloberfläche entspricht. Diese kann dann insbesondere zum Führen eines herkömmlichen, mit einem kugeligen Kopfes versehenen Acetabulum-Fräsers genutzt werden.

Das Acetabulum lässt sich besonders gut und sicher bearbeiten, wenn die Bearbeitungsfläche eine Mehrzahl von Schneidelementen trägt oder umfasst. Insbesondere können die Schneidelemente aus demselben Material gebildet sein wie der Fräskopf oder aus einem anderen Material, welches zur Bearbeitung von Knochen vorteilhafte Eigenschaften aufweist.

Günstig ist es, wenn der Fräskopf eine in distaler Richtung weisende Fräskopfanschlagfläche aufweist. Diese kann insbesondere mit der in proximaler Richtung weisenden Anschlagfläche am Formkörper zusammenwirken, um ein Eindringen des Fräskopfs in den Beckenknochen zu begrenzen.

Besonders einfach herstellen lässt sich das Instrumentarium, wenn die Fräskopfanschlagfläche eben oder im Wesentlichen eben ausgebildet ist und quer zur Fräserschaftlängsachse verläuft. Insbesondere kann sie so flächig an einer Anschlagfläche des Formkörpers anliegen, die eine Ebene definiert, die senkrecht zur Längsachse des Führungsschafts verläuft.

Gemäß einer weiteren bevorzugten Ausführungsform der Erfindung kann vorgesehen sein, dass der Fräskopf eine in proximaler Richtung weisende Endfläche aufweist, welche eben oder im Wesentlichen eben ausgebildet ist. Insbesondere kann die Endfläche quer zur Fräserschaftlängsachse verlaufen und eine Endflächenebene definieren, welche einen Kugelmittelpunkt der Kugeloberfläche enthält. Auf diese Weise wird der größte Durchmesser des Fräskopfs im Bereich der in proximaler Richtung weisenden Endfläche definiert.

Vorteilhaft ist es, wenn das zweite Fräswerkzeug in Form eines Acetabulum-Fräsers mit einem kugeligen Acetabulum-Fräskopf und einem in proximaler Richtung abstehenden Acetabulum-Fräserschaft ausgebildet ist. Wurde in einem ersten Schritt mit dem Eingangsfräser eine Eingangsbohrung am Acetabulum ausgebildet, kann in einem zweiten Schritt mit dem Acetabulum-Fräser das Pfannenlager in gewünschter Weise fertiggestellt werden.

Die nachfolgende Beschreibung bevorzugter Ausführungsformen der Erfindung dient im Zusammenhang mit den Zeichnungen der näheren Erläuterung. Es zeigen:
- Figur 1:: eine schematische Seitenansicht eines medizinischen Instrumentariums umfassend ein Führungsinstrument und einen Eingangsfräser;
- Figur 2:: eine schematische Ansicht des am pathologischen Acetabulum angesetzten Führungsinstruments;
- Figur 3:: eine schematische perspektivische Ansicht beim Aufsetzen des Eingangsfräsers auf den Führungsschaft des am Acetabulum angelegten Führungsinstruments;
- Figur 4:: eine schematische perspektivische Ansicht des auf das Führungs-instrument aufgesetzten Eingangsfräsers beim Fräsen einer Eingangsbohrung am Acetabulum;
- Figur 5:: eine schematische perspektivische Ansicht der Anordnung aus Figur 4 von der Seite;
- Figur 6:: eine schematische perspektivische Ansicht des am Beckenknochen angesetzten Führungsinstruments mit präparierter Eingangsbohrung nach Abziehen des Eingangsfräsers;
- Figur 7:: eine schematische perspektivische Ansicht beim Heranführen eines Acetabulum-Fräsers an die präparierte Eingangsbohrung nach Entfernen des Führungsinstruments; und
- Figur 8:: eine schematische perspektivische Ansicht des Beckens beim Präparieren des Pfannenlagers mit dem Acetabulum-Fräser.

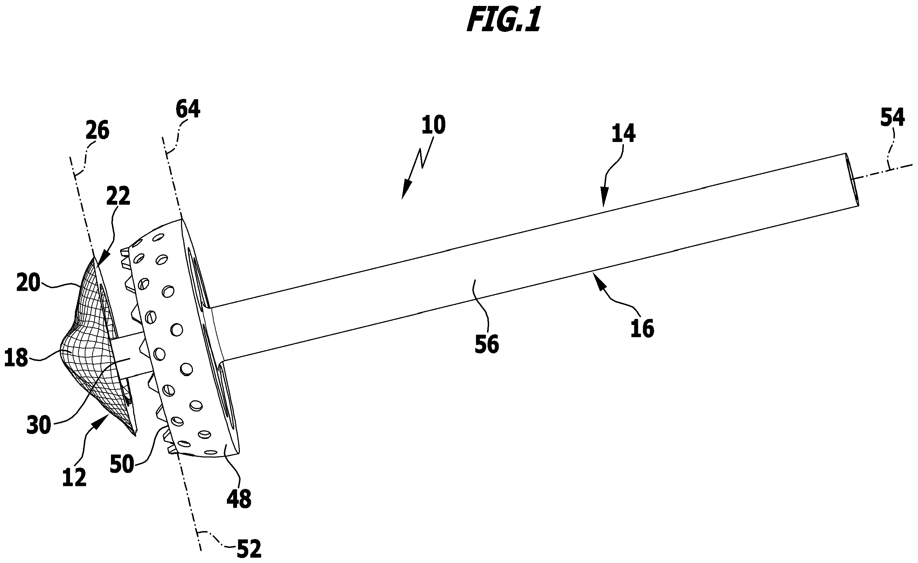

In Figur 1 ist schematisch ein insgesamt mit dem Bezugszeichen 10 bezeichnetes medizinisches Instrumentarium dargestellt. Es umfasst ein medizinisches Führungsinstrument 12 und ein erstes Fräswerkzeug 14 in Form eines Eingangs- oder Eröffnungsfräsers 16.

Das Führungsinstrument 12 umfasst einen Formkörper 18, welcher eine in distaler Richtung weisende, patientenindividuelle Knochenanlagefläche 20 aufweist.

Der Formkörper 18 ist vorzugsweise schalenförmig und durchbrechungsfrei ausgebildet und umfasst einen in distaler Richtung wirkenden Anschlag 22 für das erste Fräswerkzeug 14. Der Anschlag 22 umfasst eine in proximaler Richtung weisende Anschlagfläche 24. Diese ist eben ausgebildet und definiert eine Ebene 26, welche sich quer zu einer Längsachse 28 des Führungsinstruments 12 erstreckt. Die Längsachse 28 wird durch einen Führungsschaft 30 definiert, welcher in proximaler Richtung weisend vom Formkörper 18 absteht. Der Formkörper 18 weist vorzugsweise eine konstante oder im Wesentlichen konstante Dicke auf.

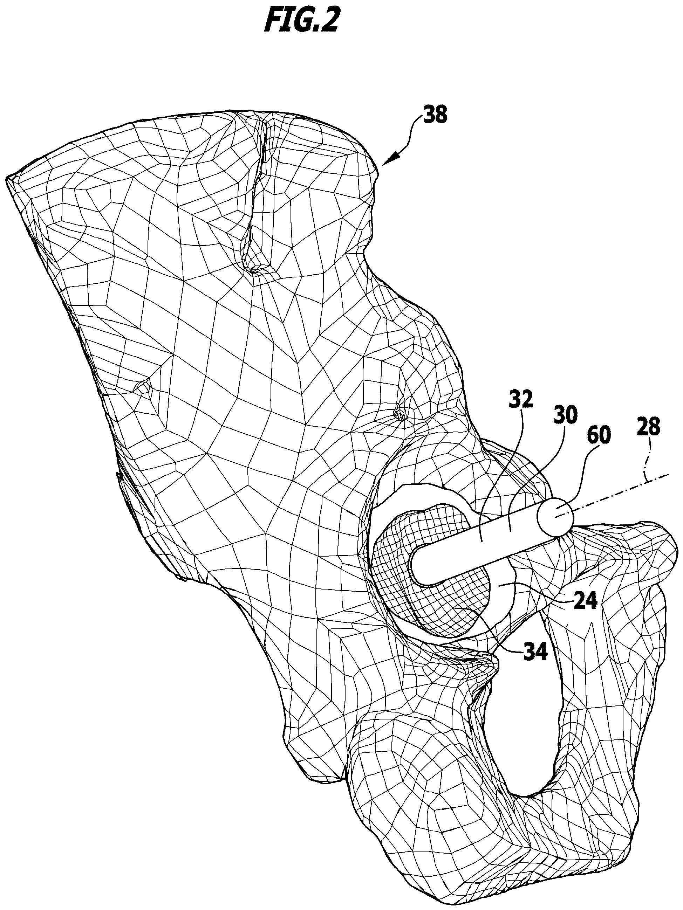

Wie beispielsweise in Figur 2 gut zu erkennen, variiert eine Breite der Anschlagfläche 24 in Abhängigkeit eines Umfangswinkels. Mit anderen Worten ist die Anschlagfläche 24 nicht in Form eines zur Längsachse 28 konzentrischen Kreisrings ausgebildet, sondern in Form einer Ringfläche, die sowohl innen als auch außen von einer unregelmäßigen Begrenzungslinie begrenzt ist. Ferner ist ein Übergang von der Knochenanlagefläche 20 zur an sie angrenzenden Anschlagfläche 24 tangentenunstetig.

Der Führungsschaft 30 ist in Form eines langgestreckten, zylindrischen Stabs 32 ausgebildet. Er erstreckt sich von einer in proximaler Richtung weisenden Seitenfläche 34 des Formkörpers 18 weg.

Die Knochenanlagefläche 20 ist korrespondierend zu einer Knochenoberfläche 36 des Beckenknochens 38 des Patienten in dem Bereich geformt, wo das Pfannenlager 40 für die Pfanne der zu implantierenden Hüftgelenkendoprothese platziert werden soll. Damit ist die Knochenanlagefläche 20 an eine Kontur des Acetabulums 42 des Patienten angepasst. Sie weist insbesondere eine Kontur auf, welche der Fossa 44, mindestens einem Teil derselben und/oder der Incisura Acetabuli 46 oder einem Teil derselben entspricht.

Die Knochenanlagefläche 20 am Formkörper 18 und/oder der Formkörper 18 sind vorzugsweise durch Gießen oder 3D-Drucken hergestellt. Zum Gießen wird eine Gießform auf Basis nichtinvasiv bestimmter Knochenkonturdaten des Patienten ausgebildet. Beispielsweise können die Knochenkonturdaten aus Röntgen- und/oder Magnetresonanzaufnahmen des Acetabulums 42 des Patienten stammen. Beispielsweise können direkt digitalisierte Röntgenaufnahmen weiter verarbeitet werden zu Druckdaten für einen 3D-Drucker, mit dem dann die Knochenanlagefläche 20 auf einem Träger beziehungsweise als Teil des Formkörpers 18 mit diesem zusammen gedruckt werden kann, optional auch zusammen mit dem Führungsschaft 30. Auf diese Weise kann dann eine Knochenanlagefläche 20 ausgebildet werden, die Knochenanlageflächenkonturdaten definiert, die den nichtinvasiv bestimmten Knochenkonturdaten des Patienten entsprechen.

Das Führungsinstrument 12 dient zum Führen des Eingangsfräsers 16. Dieser weist einen Fräskopf 48 auf mit einer in distaler Richtung weisenden Fräskopfanschlagfläche 50. Diese ist vorzugsweise eben und definiert eine Anschlagebene 52, die quer, insbesondere senkrecht, zu einer Fräserschaftlängsachse 54 verläuft, welche durch einen Fräserschaft 56 des Eingangsfräsers 16 definiert wird. Der Fräserschaft 56 ist kanuliert ausgebildet und weist eine sich konzentrisch zur Fräserschaftlängsachse 54 verlaufende Durchgangsbohrung 58 auf, deren Innendurchmesser an einen Außendurchmesser des Führungsschafts 30 angepasst ist und diesem vorzugsweise im Wesentlichen entspricht. Diese Ausgestaltung ermöglicht es, den Eingangsfräser 16 von proximal her kommend über ein proximales Ende 60 des Führungsschafts 30 auf diesen aufzuschieben.

Ferner weist der Fräskopf 48 eine in proximaler Richtung weisende Endfläche 62 auf, welche vorzugsweise eben ausgebildet ist und eine Endflächenebene 64 definiert, welche quer, insbesondere senkrecht, zur Fräserschaftlängsachse 54 verläuft. Der Fräskopf 48 weist ferner eine ringförmig in sich geschlossene Bearbeitungsfläche 66 auf, die einen Ausschnitt einer Kugeloberfläche bildet. Die Bearbeitungsfläche 66 trägt eine Mehrzahl von Schneidelementen 80, die beispielsweise in Form von in radialer Richtung von der Bearbeitungsfläche 66 abstehenden Fräszähnen ausgebildet sind. Optional ist die Endfläche 62 derart gestaltet, dass ein Kugelmittelpunkt der durch die Bearbeitungsfläche 66 definierten Kugeloberfläche auf der Endflächenebene 64 liegt.

Das Instrumentarium 10 umfasst ferner ein zweites Fräswerkzeug 68, welches in Form eines Acetabulum-Fräsers 70 ausgebildet ist. Dieser weist einen im Wesentlichen kugeligen Acetabulum-Fräskopf 72 auf und einen von diesem in proximaler Richtung abstehenden, eine Acetabulum-Fräser-Längsachse 74 definierenden, langgestreckten Acetabulum-Fräserschaft 76. Der AcetabulumFräser 70 kann insbesondere in Form eines herkömmlichen Acetabulum-Fräsers ausgebildet sein.

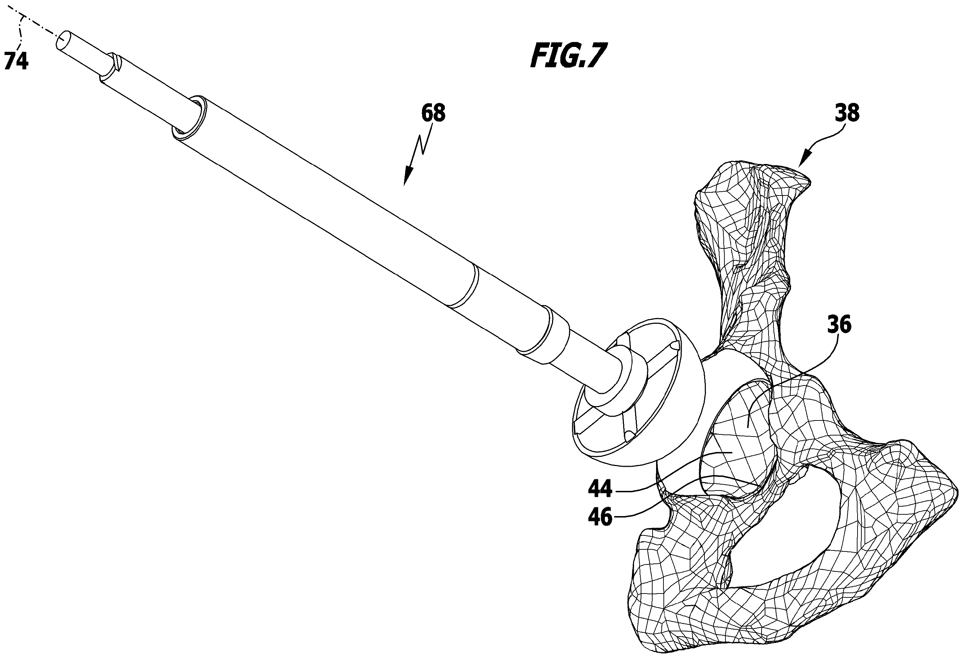

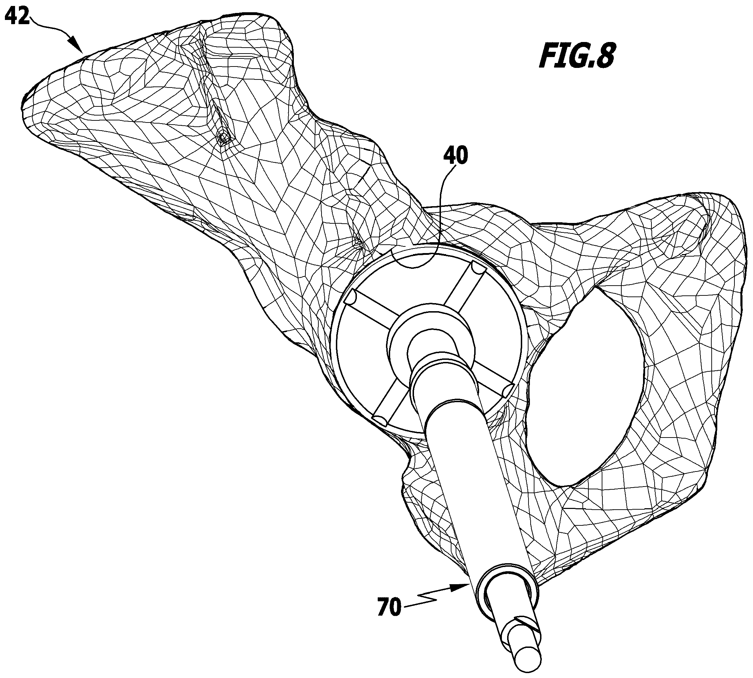

Die Funktionsweise des Instrumentariums 10 sowie die Vorgehensweise bei der Präparation des geplanten Pfannenlagers 40 wird in Verbindung mit den Figuren 2 bis 8 nachfolgend näher erläutert.

Das auf Basis der patientenindividuellen Knochenkonturdaten hergestellte Führungsinstrument 12 wird mit dem Formkörper 18 voran in den Patientenkörper eingeführt und an die korrespondierende Knochenoberfläche 36 vollflächig angelegt. Dieser Schritt ist in Figur 2 schematisch dargestellt.

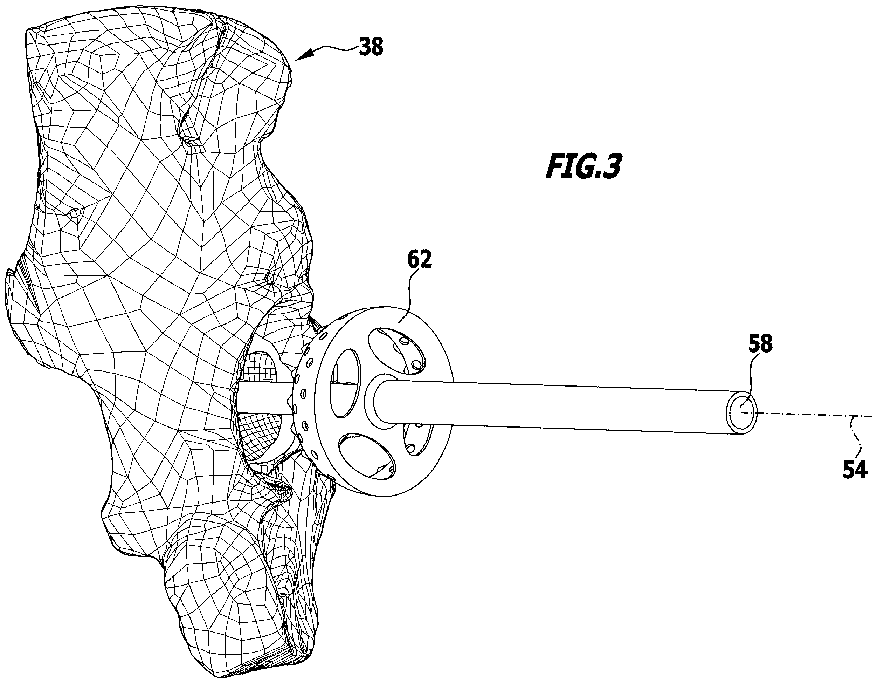

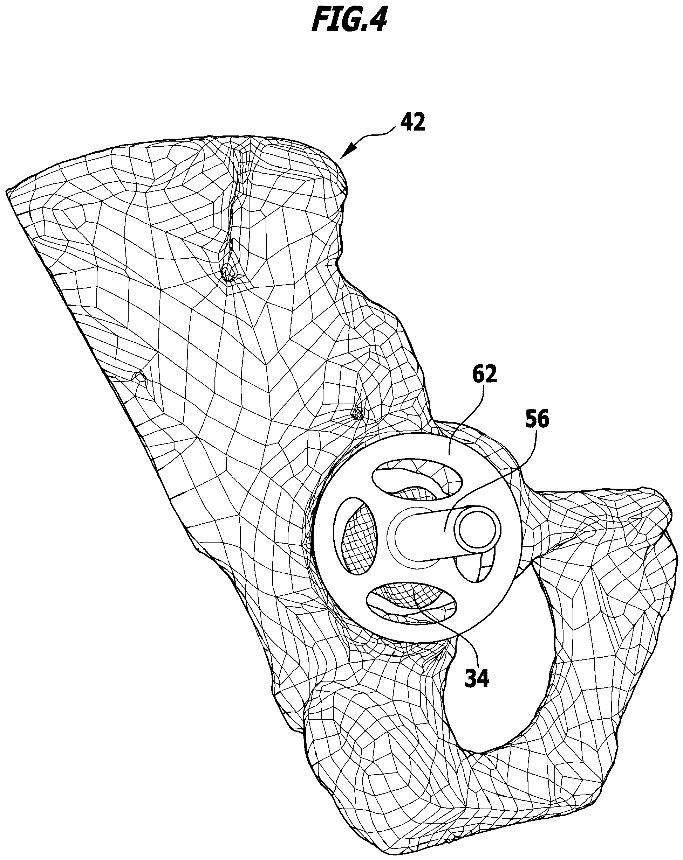

Als nächstes wird der Eingangsfräser 16 mit seinem kanulierten Fräserschaft 56 auf den Führungsschaft 30 des Führungsinstruments 12 aufgeschoben und der Fräskopf 48 an den Beckenknochen 38 herangeführt. Dies ist beispielhaft in den Figuren 3 und 4 dargestellt.

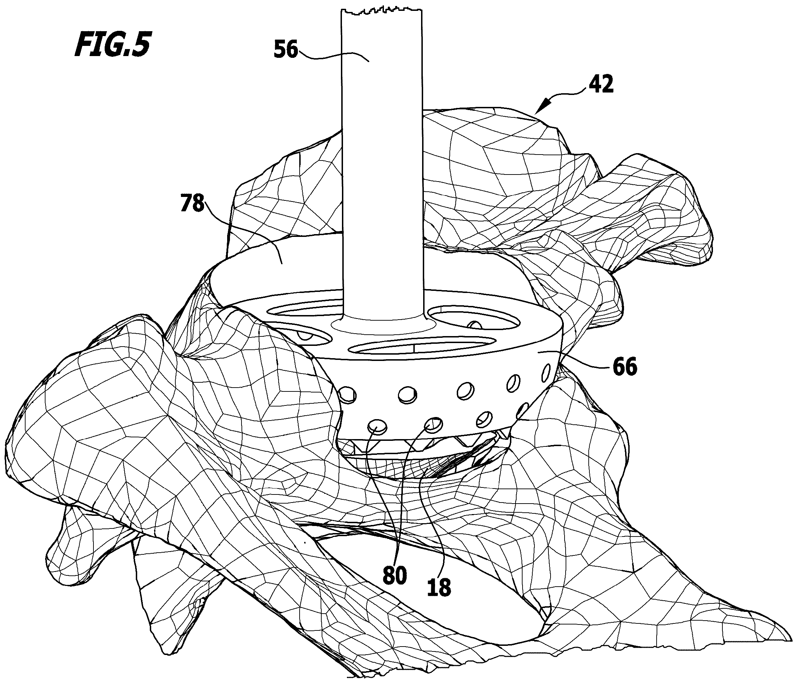

Nun kann der Eingangsfräser 16 mit einem geeigneten Antrieb in Rotation versetzt werden, um eine Eingangsbohrung 78 am Acetabulum 42 zu präparieren. Die Eingangsbohrung 78 weist eine Form auf, die einem hohlkugeligen Flächenbereich entspricht, welcher im Wesentlichen durch die Bearbeitungsfläche 66 definiert wird. Dieser Schritt ist insbesondere in Figur 5 schematisch dargestellt.

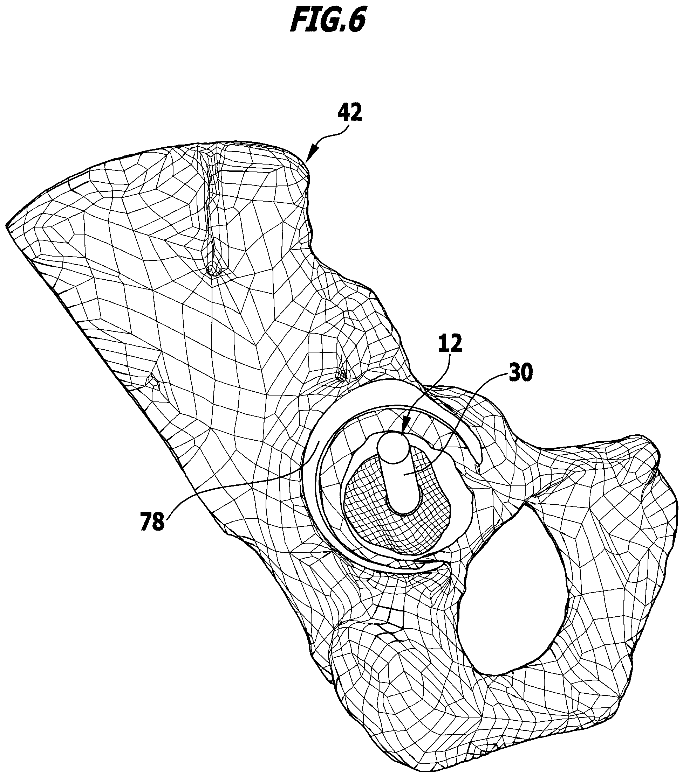

Nach der Herstellung der Eingangsbohrung 78 kann der Eingangsfräser 16 wieder abgezogen werden. Dieser Verfahrensschritt ist in Figur 6 schematisch dargestellt.

Nun können auch das Führungsinstrument 12 entfernt und mit dem Acetabulum-Fräser 70 das eigentliche Pfannenlager 40 präpariert werden. Dieses weist dann eine im Wesentlichen hohlkugelige Form auf und dient der Aufnahme der Pfanne der Hüftgelenkendoprothese.

Das Führungsinstrument 12 kann ganz oder teilweise aus einem sterilisierbaren Kunststoff oder einem Metall hergestellt sein.

Optional ist es auch möglich, den Führungsschaft 30 und den Formkörper 18 separat voneinander auszubilden und eine entsprechende Kopplungseinrichtung vorzusehen, um diese beiden Teile miteinander zu verbinden. Diese optionale Ausgestaltung ermöglicht es beispielsweise, den Führungsschaft 30 aus einem Metall herzustellen und wieder zu verwenden, so dass nur der Formkörper 18 mit einem entsprechenden Kopplungselement zum Verbinden mit dem Führungsschaft 30 gegossen oder gedruckt werden muss. Die Kopplungselemente können insbesondere in Form von Vorsprüngen oder korrespondierenden Aufnahmen ausgebildet sein, die beispielsweise mit zueinander korrespondierenden Innen- und Außengewindeabschnitten versehen sein können.

### Bezugszeichenliste

- 10:: Instrumentarium
- 12:: Führungsinstrument
- 14:: Erstes Fräswerkzeug
- 16:: Eingangsfräser
- 18:: Formkörper
- 20:: Knochenanlagefläche
- 22:: Anschlag
- 24:: Anschlagfläche
- 26:: Ebene
- 28:: Längsachse
- 30:: Führungsschaft
- 32:: Stab
- 34:: Seitenfläche
- 36:: Knochenoberfläche
- 38:: Beckenknochen
- 40:: Pfannenlager
- 42:: Acetabulum
- 44:: Fossa
- 46:: Incisura Acetabuli
- 48:: Fräskopf
- 50:: Fräskopfanschlagfläche
- 52:: Anschlagebene
- 54:: Fräserschaftlängsachse
- 56:: Fräserschaft
- 58:: Durchgangsbohrung
- 60:: Ende
- 62:: Endfläche
- 64:: Endflächenebene
- 66:: Bearbeitungsfläche
- 68:: Zweites Fräswerkzeug
- 70:: Acetabulum-Fräser
- 72:: Acetabulum-Fräskopf
- 74:: Acetabulum-Fräser-Längsachse
- 76:: Acetabulum-Fräserschaft
- 78:: Eingangsbohrung
- 80:: Schneidelement

## Patentansprüche

1. Medizinisches Instrumentarium (10) zum Implantieren einer Hüftgelenkpfanne umfassend mindestens ein medizinisches Führungsinstrument (12) für ein erstes Fräswerkzeug (14), wobei das Führungsinstrument (12) einen Formkörper (18) umfasst, welcher Formkörper (18) eine patientenindividuelle, in distaler Richtung weisende und von einem Kugeloberflächenausschnitt abweichende Knochenanlagefläche (20) aufweist, welche Knochenanlagefläche (20) korrespondierend oder im Wesentlichen korrespondierend zu einer Knochenoberfläche (36) des Patienten geformt ist, wobei vom Formkörper (18) in proximaler Richtung weisend ein eine Längsachse (28) definierender Führungsschaft (30) zum Führen des ersten Fräswerkzeugs (14) absteht und wobei das medizinische Instrumentarium ein erstes Fräswerkzeug (14) umfasst, **dadurch gekennzeichnet, dass** das erste Fräswerkzeug (14) in Form eines Eingangsfräsers (16) ausgebildet ist mit einem kanulierten, eine Fräserschaftlängsachse (54) definierenden Fräserschaft (56), welcher ausgebildet ist zur Aufnahme des Führungsschafts (30) des medizinischen Führungsinstruments (12).

2. Medizinisches Instrumentarium nach Anspruch 1, **dadurch gekennzeichnet,**
a) **dass** die patientenindividuelle Knochenanlagefläche (20) in distaler Richtung weisend angeordnet oder ausgebildet ist und dass der Führungsschaft (30) in proximaler Richtung weisend angeordnet oder ausgebildet ist
und/oder
b) **dass** der Formkörper (18) einen in distaler Richtung wirkenden Anschlag (22) für ein Fräswerkzeug (14) aufweist.

3. Medizinisches Instrumentarium nach Anspruch 2, **dadurch gekennzeichnet, dass** der mindestens eine Anschlag (22) einen in proximaler Richtung weisende Anschlagfläche (24) aufweist.

4. Medizinisches Instrumentarium nach Anspruch 3, **dadurch gekennzeichnet,**
a) **dass** die Anschlagfläche (24) eben oder im Wesentlichen eben ausgebildet ist
und/oder
b) **dass** die Anschlagfläche (24) eine Ebene (26) definiert, welche quer, insbesondere senkrecht, zur Längsachse (28) verläuft
und/oder
c) **dass** die Anschlagfläche (24) an die patientenindividuelle Knochenanlagefläche (20) angrenzt und dass ein Übergang zwischen der Anschlagfläche (24) und der patientenindividuellen Knochenanlagefläche (20) tangentenunstetig ist
und/oder
d) **dass** eine Breite der Anschlagfläche (24) in Abhängigkeit eines Umfangswinkels variiert.

5. Medizinisches Instrumentarium nach einem der voranstehenden Ansprüche, **dadurch gekennzeichnet,**
a) **dass** die Knochenanlagefläche (20) mindestens teilweise an eine Kontur des Acetabulums (42) des Patienten angepasst ist
und/oder
b) **dass** die Knochenanlagefläche (20) eine Kontur aufweist, welche mindestens einem Teil der Fossa (44) und/oder einem Teil der Incisura Acetabuli (46) entspricht.

6. Medizinisches Instrumentarium nach einem der voranstehenden Ansprüche, **dadurch gekennzeichnet, dass** der Formkörper (18) schalenförmig ausgebildet ist und eine konstante oder im Wesentlichen konstante Dicke aufweist.

7. Medizinisches Instrumentarium nach einem der voranstehenden Ansprüche, **dadurch gekennzeichnet,**
a) **dass** die Knochenanlagefläche (20) und/oder der Formkörper (18) durch Gießen oder 3D-Drucken hergestellt ist
und/oder
b) **dass** die Knochenanlagefläche (20) des Formkörpers (18) Knochenanlageflächenkonturdaten definiert, welche nichtinvasiv bestimmten Knochenkonturdaten des Patienten entsprechen oder im Wesentlichen entsprechen, insbesondere Knochenkonturdaten des Patienten aus Röntgen- oder Magnetresonanzaufnahmen.

8. Medizinisches Instrumentarium nach einem der voranstehenden Ansprüche, **dadurch gekennzeichnet,**
a) **dass** der Führungsschaft (30) unlösbar oder lösbar verbindbar mit dem Formkörper (18) verbunden ist
und/oder
b) **dass** das medizinische Instrumentarium eine Kopplungseinrichtung zum temporären Koppeln des Formkörpers (18) mit dem Führungsschaft (30) umfasst.

9. Medizinisches Instrumentarium nach Anspruch 8, **dadurch gekennzeichnet, dass** die Kopplungseinrichtung mindestens ein erstes Kopplungselement und mindestens ein zweites Kopplungselement umfasst und dass die mindestens einen ersten und zweiten Kopplungselemente korrespondierend zueinander ausgebildet sind und in einer Kopplungsstellung in Eingriff stehen und in einer Lösestellung außer Eingriff stehen.

10. Medizinisches Instrumentarium nach Anspruch 9, **dadurch gekennzeichnet,**
a) **dass** das mindestens eine erste Kopplungselement in Form einer Kopplungsaufnahme und das mindestens eine zweite Kopplungselement in Form eines Kopplungsvorsprungs ausgebildet sind
und/oder
b) **dass** das mindestens eine erste Kopplungselement einen Innengewindeabschnitt und das mindestens eine zweite Kopplungselement einen korrespondierenden Außengewindeabschnitt umfasst
und/oder
c) **dass** das mindestens eine erste Kopplungselement am Formkörper (18) angeordnet oder ausgebildet ist und dass das mindestens eine zweite Kopplungselement am Führungsschaft (30) angeordnet oder ausgebildet ist.

11. Medizinisches Instrumentarium nach einem der voranstehenden Ansprüche, **dadurch gekennzeichnet, dass** der Führungsschaft (30) in Form eines langgestreckten zylindrischen Stabes (32) ausgebildet ist.

12. Medizinisches Instrumentarium nach einem der voranstehenden Ansprüche, **gekennzeichnet durch** ein zweites Fräswerkzeug (68).

13. Medizinisches Instrumentarium nach einem der voranstehenden Ansprüche, **dadurch gekennzeichnet, dass** der Eingangsfräser (16) einen Fräskopf (48) aufweist und dass der Fräskopf (48) eine in sich geschlossene Bearbeitungsfläche (66) aufweist, dass die Bearbeitungsfläche (66) einen Ausschnitt einer Kugeloberfläche bildet.

14. Medizinisches Instrumentarium nach Anspruch 13, **dadurch gekennzeichnet,**
a) **dass** die Bearbeitungsfläche (66) eine Mehrzahl von Schneidelementen (80) trägt oder umfasst
und/oder
b) **dass** der Fräskopf (48) eine in distaler Richtung weisende Fräskopfanschlagfläche (50) aufweist,
wobei insbesondere die Fräskopfanschlagfläche (50) eben oder im Wesentlichen eben ausgebildet ist und quer zur Fräserschaftlängsachse (54) verläuft
und/oder
c) **dass** der Fräskopf (48) eine in proximaler Richtung weisende Endfläche (62) aufweist, welche eben oder im Wesentlichen eben ausgebildet ist und insbesondere quer zur Fräserschaftlängsachse (54) verläuft und insbesondere eine Endflächenebene (64) definiert, welche einen Kugelmittelpunkt der Kugeloberfläche enthält.

15. Medizinisches Instrumentarium nach einem der Ansprüche 12 bis 14, **dadurch gekennzeichnet, dass** das zweite Fräswerkzeug (68) in Form eines Acetabulum-Fräsers (70) mit einem kugeligen Acetabulum-Fräskopf (72) und einem in proximaler Richtung abstehenden Acetabulum-Fräserschaft (76) ausgebildet ist.

## Claims

1. Medical instrumentation (10) for implanting an acetabular cup, comprising at least one medical guiding instrument (12) for a first reaming tool (14), wherein the guiding instrument (12) comprises a shaped body (18), which shaped body (18) has a patient-specific bone abutment surface (20) facing in the distal direction and deviating from a spherical surface section, which bone abutment surface (20) is shaped so as to correspond or substantially correspond to a bone surface (36) of the patient, wherein a guiding shaft (30), defining a longitudinal axis (28), for guiding the first reaming tool (14) protrudes from the shaped body (18) and faces in the proximal direction and wherein the medical instrumentation comprises a first reaming tool (14), **characterized in that** the first reaming tool (14) is constructed in the form of an entrance reamer (16) with a cannulated reamer shaft (56) which defines a reamer shaft longitudinal axis (54), which reamer shaft (56) is constructed to receive the guiding shaft (30) of the medical guiding instrument (12).

2. Medical instrumentation in accordance with claim 1, **characterized**
a) **in that** the patient-specific bone abutment surface (20) is arranged or constructed so as to face in the distal direction, and in that the guiding shaft (30) is arranged or constructed so as to face in the proximal direction
and/or
b) **in that** the shaped body (18) has a stop (22) acting in the distal direction for a reaming tool (14).

3. Medical instrumentation in accordance with claim 2, **characterized in that** the at least one stop (22) has a stop surface (24) facing in the proximal direction.

4. Medical instrumentation in accordance with claim 3, **characterized**
a) **in that** the stop surface (24) is of flat or substantially flat construction
and/or
b) **in that** the stop surface (24) defines a plane (26) which extends transversely, in particular, perpendicularly, to the longitudinal axis (28)
and/or
c) **in that** the stop surface (24) adjoins the patient-specific bone abutment surface (20), and in that a transition between the stop surface (24) and the patient-specific bone abutment surface (20) is tangentially discontinuous
and/or
d) **in that** a width of the stop surface (24) varies as a function of a circumferential angle.

5. Medical instrumentation in accordance with any one of the preceding claims, **characterized in that**
a) the bone abutment surface (20) is at least partially adapted to a contour of the acetabulum (42) of the patient
and/or
b) **in that** the bone abutment surface (20) has a contour which corresponds at least to a portion of the fossa (44) and/or to a portion of the acetabular notch (46).

6. Medical instrumentation in accordance with any one of the preceding claims, **characterized in that** the shaped body (18) is of dish-shaped construction and has a constant or substantially constant width.

7. Medical instrumentation in accordance with any one of the preceding claims, **characterized**
a) **in that** the bone abutment surface (20) and/or the shaped body (18) are manufactured by molding or 3-D printing
and/or
b) **in that** the bone abutment surface (20) of the shaped body (18) defines bone abutment surface contour data which correspond or substantially correspond to noninvasively determined bone contour data of the patient, in particular, bone contour data of the patient from X-ray or magnetic resonance images.

8. Medical instrumentation in accordance with any one of the preceding claims, **characterized**
a) **in that** the guiding shaft (30) is connected to the shaped body (18) in a non-releasably or releasably connectable manner
and/or
b) **in that** the medical instrumentation comprises a coupling device for temporarily coupling the shaped body (18) to the guiding shaft (30).

9. Medical instrumentation in accordance with claim 8, **characterized in that** the coupling device comprises at least one first coupling element and at least one second coupling element, and **in that** the at least one first and second coupling elements are constructed so that they correspond to each other and are in engagement in a coupling position and in disengagement in a release position.

10. Medical instrumentation in accordance with claim 9, **characterized in that**
a) the at least one first coupling element is constructed in the form of a coupling receptacle and the at least one second coupling element in the form of a coupling projection
and/or
b) the at least one first coupling element has an internal thread section and the at least one second coupling element has a corresponding external thread section
and/or
c) the at least one first coupling element is arranged or constructed on the shaped body (18), and the at least one second coupling element is arranged or constructed on the guiding shaft (30).

11. Medical instrumentation in accordance with any one of the preceding claims, **characterized in that** the guiding shaft (30) is constructed in the form of an elongate cylindrical rod (32).

12. Medical instrumentation in accordance with any one of the preceding claims, **characterized by** a second reaming tool (68).

13. Medical instrumentation in accordance with any one of the preceding claims, **characterized in that** the entrance reamer (16) has a reamer head (48), **in that** the reamer head (48) has a machining surface (66) closed within itself, **in that** the machining surface (66) forms a section of a spherical surface.

14. Medical instrumentation in accordance with claim 13, **characterized**
a) **in that** the machining surface (66) carries or comprises a plurality of cutting elements (80)
and/or
b) **in that** the reamer head (48) has a reamer head stop surface (50) facing in the distal direction,
wherein, in particular, the reamer head stop surface (50) is of flat or substantially flat construction and extends transversely to the reamer shaft longitudinal axis (54)
and/or
c) **in that** the reamer head (48) has an end face (62) facing in the proximal direction, which is of flat or substantially flat construction and, in particular, extends transversely to the reamer shaft longitudinal axis (54) and, in particular, defines an end face plane (64) containing a sphere center of the spherical surface.

15. Medical instrumentation in accordance with any one of claims 12 to 14, **characterized in that** the second reaming tool (68) is constructed in the form of an acetabulum reamer (70) with a spherical acetabulum reamer head (72) and an acetabulum reamer shaft (76) protruding in the proximal direction.

## Revendications

1. Instrumentation médicale (10) destinée à l'implantation d'une cupule d'articulation de la hanche, comprenant au moins un instrument de guidage médical (12) pour un premier outil de fraisage (14), instrumentation médicale
dans laquelle l'instrument de guidage (12) comporte un corps de forme (18), ce corps de forme (18) présentant une surface d'appui sur l'os (20) individuelle pour chaque patient, dirigée en direction distale et s'écartant d'une surface de secteur sphérique, et la surface d'appui sur l'os (20) étant formée de manière correspondante ou sensiblement correspondante à une surface de l'os (36) du patient,
dans laquelle du corps de forme (18) fait saillie, en étant dirigée en direction proximale, une tige de guidage (30) définissant un axe longitudinal (28) et destiné à guider le premier outil de fraisage (14), et dans laquelle l'instrumentation médicale comprend un premier outil de fraisage (14),
**caractérisée en ce que** le premier outil de fraisage (14) est réalisé sous forme de fraise d'entrée (16) comportant un corps allongé de fraise (56) canulé et définissant un axe longitudinal (54) du corps allongé de fraise, qui est conçu pour accueillir la tige de guidage (30) de l'instrument de guidage médical (12).

2. Instrumentation médicale selon la revendication 1, **caractérisée**
a) **en ce que** la surface d'appui sur l'os (20) individuelle du patient, est agencée ou réalisée de manière à être dirigée en direction distale, et en ce que la tige de guidage (30) est agencée ou réalisée de manière à être dirigée en direction proximale,
et/ou
b) **en ce que** le corps de forme (18) présente une butée (22) agissant en direction distale, pour un outil de fraisage (14).

3. Instrumentation médicale selon la revendication 2, **caractérisée en ce que** ladite au moins une butée (22) présente une surface de butée (24) dirigée en direction proximale.

4. Instrumentation médicale selon la revendication 3, **caractérisée**
a) **en ce que** la surface de butée (24) est d'une configuration plane ou sensiblement plane,
et/ou
b) **en ce que** la surface de butée (24) définit un plan (26) qui s'étend transversalement, de préférence perpendiculairement, à l'axe longitudinal (28),
et/ou
c) **en ce que** la surface de butée (24) est adjacente à la surface d'appui sur l'os (20) individuelle du patient, et en ce qu'une transition entre la surface de butée (24) et la surface d'appui sur l'os (20) individuelle du patient, présente une discontinuité tangentielle,
et/ou
d) **en ce qu'**une largeur de la surface de butée (24) varie en fonction d'un angle circonférentiel.

5. Instrumentation médicale selon l'une des revendications précédentes, **caractérisée**
a) **en ce que** la surface d'appui sur l'os (20) est adaptée au moins en partie à un contour de l'acétabulum (42) du patient,
et/ou
b) **en ce que** la surface d'appui sur l'os (20) présente un contour qui correspond au moins à une partie de la fosse acétabulaire (44) et/ou de l'incisure de l'acétabulum (46).

6. Instrumentation médicale selon l'une des revendications précédentes, **caractérisée en ce que** le corps de forme (18) est en forme de coque est présente une épaisseur constante ou sensiblement constante.

7. Instrumentation médicale selon l'une des revendications précédentes, **caractérisée**
a) **en ce que** la surface d'appui sur l'os (20) et/ou le corps de forme (18) est fabriqué par moulage ou par impression 3D,
et/ou
b) **en ce que** la surface d'appui sur l'os (20) du corps de forme (18) définit des données de contour de surface d'appui sur l'os, qui correspondent ou correspondent sensiblement à des données de contour d'os du patient ayant été déterminées de manière non invasive, notamment des données de contour d'os du patient provenant de clichés de radiographie ou de résonance magnétique.

8. Instrumentation médicale selon l'une des revendications précédentes, **caractérisée**
a) **en ce que** la tige de guidage (30) est reliée de manière indémontable ou démontable au corps de forme (18),
et/ou
b) **en ce que** l'instrumentation médicale comprend un dispositif de couplage pour assurer un couplage temporaire du corps de forme (18) avec la tige de guidage (30).

9. Instrumentation médicale selon la revendication 8, **caractérisée en ce que** le dispositif de couplage comprend au moins un premier élément de couplage et au moins un deuxième élément de couplage, et **en ce que** lesdits au moins un premier et un deuxième éléments de couplage sont d'une configuration mutuellement correspondante, et sont en prise réciproque dans une position couplée et hors de prise dans une position détachée.

10. Instrumentation médicale selon la revendication 9, **caractérisée**
a) **en ce que** ledit au moins un premier élément de couplage est réalisé sous la forme d'un logement de couplage et ledit au moins un deuxième élément de couplage est réalisé sous la forme d'une protubérance de couplage,
et/ou
b) **en ce que** ledit au moins un premier élément de couplage comporte un tronçon de filetage intérieur et ledit au moins un deuxième élément de couplage comporte un tronçon de filetage extérieur correspondant,
et/ou
c) **en ce que** ledit au moins un premier élément de couplage est agencé ou formé sur le corps de forme (18), et en ce que ledit au moins un deuxième élément de couplage est agencé ou formé sur la tige de guidage (30).

11. Instrumentation médicale selon l'une des revendications précédentes, **caractérisée en ce que** la tige de guidage (30) est réalisée sous la forme d'une barre cylindrique (32) allongée.

12. Instrumentation médicale selon l'une des revendications précédentes, **caractérisée par** un deuxième outil de fraisage (68).

13. Instrumentation médicale selon l'une des revendications précédentes, **caractérisée en ce que** la fraise d'entrée (16) comporte une tête de fraisage (48), et **en ce que** la tête de fraisage (48) présente une surface d'usinage (66) fermée en soi, **en ce que** la surface d'usinage (66) forme un secteur de surface sphérique.

14. Instrumentation médicale selon la revendication 13, **caractérisée**
a) **en ce que** la surface d'usinage (66) porte ou comprend une pluralité d'éléments de coupe (80),
et/ou
b) **en ce que** la tête de fraisage (48) présente une surface de butée de tête de fraisage (50) dirigée en direction distale,
la surface de butée de tête de fraisage (50) étant notamment d'une configuration plane ou sensiblement plane, et s'étendant transversalement à l'axe longitudinal (54) du corps allongé de fraise,
et/ou
c) **en ce que** la tête de fraisage (48) présente une surface d'extrémité (62), qui est d'une configuration plane ou sensiblement plane et s'étend notamment transversalement à l'axe longitudinal (54) du corps allongé de fraise, et définit notamment un plan de surface d'extrémité (64), qui contient un centre de sphère de la surface sphérique.

15. Instrumentation médicale selon l'une des revendications 12 à 14, **caractérisée en ce que** le deuxième outil de fraisage (68) est réalisé sous la forme d'une fraise d'acétabulum (70) avec une tête de fraisage d'acétabulum (72) sphérique, et un corps allongé de fraise d'acétabulum (76) faisant saillie en direction proximale.
